Europäisches Patentamt

European Patent Office

Office européen des brevets

(11) Publication number: **0 318 030**
**A1**

## (12) EUROPEAN PATENT APPLICATION

(21) Application number: **88119676.0**

(22) Date of filing: **25.11.88**

(51) Int. Cl.4: **A61K 37/02 , //A61K35/38**

Claims for the following Contracting State: ES.

(30) Priority: **26.11.87 JP 298458/87**

(43) Date of publication of application:
**31.05.89 Bulletin 89/22**

(84) Designated Contracting States:
**CH DE ES FR GB IT LI NL SE**

(71) Applicant: **OTSUKA PHARMACEUTICAL CO., LTD.**
**9, Kandatsukasacho 2-chome**
**Chiyoda-ku Tokyo 101(JP)**

(72) Inventor: **Adachi, Masakazu**
**No. 3493-9, Ishihara-machi**
**Takasaki-shi Gunma(JP)**

(74) Representative: **Hansen, Bernd, Dr.rer.nat. et al**
**Hoffmann, Eitle & Partner Patentanwälte**
**Arabellastrasse 4 Postfach 81 04 20**
**D-8000 München 81(DE)**

(54) Glycoprotein, preparation method, pharmaceutical compositions comprising it and use.

(57) A glycoprotein JI-36 originating from a cancer cell membrane component, which has a highly immunogenic glyco-related antigen capable of establishing an immune response specific to cancers, is disclosed. The JI-36 is highly useful as an anticancer agent in the treatment and prevention of cancers. Pharmaceutical compositions and preparation methods are included.

EP 0 318 030 A1

# GLYCOPROTEIN, PREPARATION METHOD, PHARMACEUTICAL COMPOSITIONS COMPRISING IT AND USE

## FIELD OF THE INVENTION

This invention relates to a glycoprotein. More particularly, it relates to a novel glycoprotein which has an antigen structure comprising sugar chains specific to cancers and which can be used for a specific immunotherapy of cancer.

## BACKGROUND OF THE INVENTION

The present inventor has conducted extensive studies on an immune response of a host against cancer, and the application of the immune response to the treatment of cancer. (The term "cancer" as used herein means, in a broad sense, malignant tumors as well as diseases caused by such tumors.) During the studies, the present inventor has previously isolated a glycorelated antigen having a high immunogenicity which serves as an immunogen in a host and establishes an immune response specific to cancers from a cancer cell membrane component, and found that this antigen is highly effective for the treatment and prevention of cancers, as disclosed in Japanese Patent Application (OPI) Nos. 1420/84, 67224/84, 22519/84, 78918/85 and 214737/85. (The term "OPI" as used herein means a published unexamined Japanese patent application.)

As a result of further studies, the present inventor has found that a specific glycoprotein derived from a cancer cell membrane component contains a glycorelated antigen having a high immunogenicity which establishes an immune response specific to cancers and is stable, and that the above glycoprotein is highly useful as a pharmaceutical agent. Based on the above finding, the inventor completed the present invention.

## SUMMARY OF THE INVENTION

An object of the present invention is to provide a glycoprotein JI-36 which is derived from a cancer cell membrane component and has the following properties:

(1) it has sugar chains of a $Gal\beta1 \rightarrow 3GalNAc$ structure and a $Gal\beta1 \rightarrow 4GlcNAc\beta1 - 6$ ($Gal\beta1 \rightarrow 3$)$GalNAc$ structure;

(2) it shows a glycoprotein ratio, i.e., (neutral sugar content + aminosugar content)/protein content, of $1.3\pm0.3$, wherein the neutral sugar content is determined by the phenol-sulfonic acid method, and the aminosugar content and the protein content are determined by the Morgan-Elson reaction method and the Lowry method, respectively;

(3) it comprises, as sugar components, Gal and GlcNAc as the main components together with Fuc, Man, GalNAc and Sia;

(4) it shows the maximum absorptions at 270 to 280 nm in the UV absorption spectrum thereof; and

(5) it induces an immune response specific to cancers.

## BRIEF DESCRIPTION OF THE DRAWING

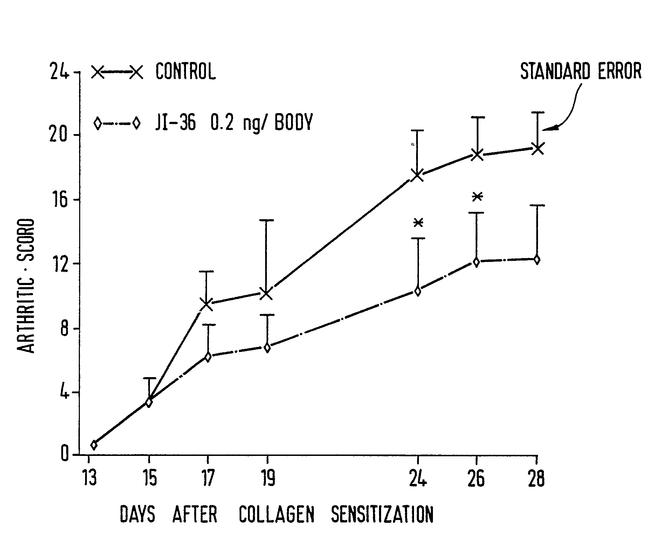

Figure 1 is a graph showing an effect of JI-36 of the present invention on the collagen-induced arthritis as compared with the control.

## DETAILED DESCRIPTION OF THE INVENTION

The abbreviations used herein are selected from among those recommended by IUPAC-IUB, those conventionally employed in the art and those which are defined herein. Examples thereof are as follows.

Gaℓ: galactose.

GaℓNAc: N-acetylgalactosamine.

GℓcNAc: N-acetylglucosamine.

Fuc: fucose.

Man: mannose.

Sia: sialic acid.

FH-2: a monoclonal antibody having a Galβ1 → 4 -(Fucαl → 3)GℓcNAc sugar chain as an epitope (cf. J. Biol. Chem., 259, 4681-4685 (1985)).

ACFH18: a monoclonal antibody having a Galβ1 → 4(Fucα1 → 3)GℓcNAcβ1 → 3Galβl → 4(Fucα1→3)-GℓcNAcβ1 → 3Galβ1 → 4(Fucα 1→3)GℓcNAc sugar chain as an epitope (cf. J. Biol. Chem., 259, 4681-4685 (1985)).

FH-6: a monoclonal antibody having a Siaα2 - 3Galβ1 → 4(Fucα1 → 3)GℓcNAcβ1 → 3Galβ1→4-(Fucα1→3)GℓcNAc sugar chain as an epitope (cf. J. Biol. Chem., 259, 10511-10517 (1984)).

AH-6: a monoclonal antibody having an Fucα1 → 2Galβ1 → 4(Fucα1 → 3)GℓcNAc sugar chain as an epitope (cf. J. Biol. Chem., 258, 11793-11797 (1983)).

PNA: peanut lectin (Arachis Hypogaea Agglutinin).

The glycoprotein JI-36 of the present invention, which is hereinafter often simply abbreviated as "JI-36", can be prepared by, for example, reducing a glycorelated antigen TCA derived from a cancer cell membrane component and then reacting the glyco-related antigen with an SH group-modifying reagent (SH reagent).

Examples of the glyco-related antigen TCA derived from a cancer cell membrane component include: (1) a glyco-related antigen isolated from a cancer cell membrane component which is capable of specifically binding to lectin which specifically binds to terminal galactose or terminal N-acetylgalactosamine as disclosed in Japanese Patent Application (OPI) No. 1420/84; (2) a glyco-related antigen isolated from a cancer cell membrane component which is capable of binding to a lectin receptor-binding antibody as disclosed in Japanese Patent Application (OPI) 67224/84; (3) a glyco-related antigen isolated from a cancer cell membrane component which has a terminal fucose sugar chain structure as disclosed in Japanese Patent Application (OPI) No. 214737/85, as well as those obtained by thermally denaturing these glyco-related antigens as disclosed in Japanese Patent Application (OPI) Nos. 225119/84, 78918/85 and 214737/85.

The reduction of the glyco-related antigen TCA can be carried out by any conventional method generally used for the cleavage of a disulfide bond of a protein. It is, however, preferably effected by using a reducing agent such as dithiothreitol (hereinafter abbreviated as DTT) or dithioerythritol.

Any conventional SH reagent can be used in the above process so long as it can modify the reduced antigen with an SH group so as not to reform the cleaved disulfide bond. Particularly preferred SH reagents include alkylating agents such as iodoacetic acid, iodoacetamide (hereinafter abbreviated as IAA), and N-ethylamaleimide, etc.

The reduction and the reaction with the SH reagent can be effected under the conditions so as not to denaturate the cancer cell membrane component as a raw material, for example, at a temperature in the range of from about 4 to about 40°C in a buffer solution, for example, a tris (tris(hydroxymethyl)-aminomethane) hydrochloride buffer (pH 8.5), etc.

In a preferred embodiment, the reduction can be carried out by adding about 20 ml of 2.5M tris hydrochloride buffer (pH 8.5) to 75 ml of crude JI-36 containing about 20 to 25 mg of proteins, and then 1 g of sodium dodecylsulfate (SDS) is added thereto. Thereafter, 5 ml of 0.5M tris hydrochloride buffer (pH 8.5) containing dithiothreitol (DTT) at a concentration of 40 mg/ml is added to the above solution, and the resulting mixture is subjected to ultrasonic treatment in a nitrogen atmosphere in a water bath at about 25°C for about 15 minutes, followed by allowing the mixture to stand at room temperature for 12 hours. Subsequent to the above reduction, 10 ml of a 0.5M tris hydrochloride buffer (pH 8.5) containing iodoacetamide (IAA) at a concentration of 40 mg/ml is added to the mixture, and then the mixture is again subjected to the ultrasonic treatment in a water bath at about 25°C for 15 minutes, followed by allowing the mixture to stand at room temperature fro 1 to 2 hours to effect alkylation.

The JI-36 of the present invention can be isolated from the reaction mixture thus obtained through various physicochemical or biochemical purifying processes by taking advantage of the properties thereof. These purification processes include, for example,, salting out, gel filtration, ultrafiltration, various chromatographic procedures such as liquid chromatography, affinity chromatography and ion exchange chromatography, electrophoresis, chromatofocusing, extraction, centrifugation, dialysis and combinations

3

EP 0 318 030 A1

thereof. Among these processes, affinity chromatography by taking advantage of the affinity of the JI-36 for PNA is particularly preferred.

The JI-36 thus obtained has the above-described properties.

In the above properties, the aminosugar content is determined by the Morgan-Elson reaction method as described in Reissig, J.L., Strominger, J.L. and Leloir, L.F., J. Biol. Chem., 217, 959 (1955), the protein content is determined by the Lowry method as described in Lowry, O.H., Rosebrough, N.J., Farr, A.L., and Randall, R.J., J. Biol. Chem., 193, 265-275 (1951), and the neutral sugar content is determined by the phenolsulfuric acid method as described in Dubois, M. et al., Anal. Chem., 28, 350 (1956).

If desired, the thus obtained JI-36 may be further heated in the same manner as in the glycorelated antigen TCA as described, for example, in Japanese Patent Application (OPI) No. 225119/84 to thereby obtain a thermally denatured product.

Further, lymphocytes may be sensitized with the JI-36 of the present invention by, for example, the same method as that used in the glyco-related antigen TCA as described in Japanese Patent Application (OPI) No. 1420/84 to thereby obtain cytotoxic lymphocytes specific to cancer cells, i.e., killer cells.

A pharmaceutical preparation containing the JI-36 of the present invention as an active ingredient is useful as an anticancer agent. This anticancer agent may be formulated into any form so long as it effectively includes the JI-36 as a main component. It is generally administered intravenously, subcutaneously or intramuscularly in the form of a liquid encapsulated in a liposome, a suspension or an emulsion. It is particularly preferred to encapsulate the preparation in a liposome, Alternately, the preparation can be in the form of a dry preparation which can be formulated into a liquid just before the use by adding an appropriate carrier thereto. These liquid preparations may further contain various additives, for example, a suspending agent such as methylcellulose, an emulsifier such as lectin, a preservative such as methyl-p-hydroxybenzoate or a stabilizer or a buffer which per se exerts no undesirable effect on the immunological functions of human or animals. An aqueous carrier such as physiological saline solution or a nonaqueous carrier such as a vegetable oil, e.g., sesame oil, a mineral oil, e.g., paraffin, an animal oil such as squalene or propylene glycol can be used. The preparation may furthermore contain an appropriate adjuvant in order to enhance the immunity. Examples of the adjuvant include Freund's complete adjuvant, saponin for animal use and aluminum hydroxide for human use.

The above described anticancer agent can be administered to a cancer patient at a single dose or at two or more multiple doses for a prolonged period of time. Further, it can be administered for prophylatic purpose to those having a risk of suffering from cancers.

Due to low toxicity of JI-36, it can be administered at a broad range of dose. Therefore, the concentration of the JI-36 in an anticancer agent is not particularly restricted. However, in general, the content of JI-36 in terms of sugars is preferably from 0.001 to 100 µg/ml. The dose of the same varies depending on the condition, age and sex of the patient to be treated. It is generally administered at a dose of from -0.001 to 1000 µg/day once or several times daily.

The JI-36 of the present invention is a glycoprotein capable of inducing an immune response specific to cancers. Thus, it is highly useful in the treatment and prevention of cancers as well as in the preparation of cytotoxic lymphocytes specific to cancers. In addition, the JI-36 of the present invention is useful in the treatment and prevention of immunopathy diseases such as rheumatoid arthritis wherein glyco-related antigen-carrying cells are observed, as reported in MINOPHAGAN MEDICAL REVIEW, 31, 184-193 (1986).

Furthermore, JI-36 shows no agglutination, and therefore, it is stable upon storage or freezing-thawing. It shows little change in the biological activity for a prolonged period of time, which makes it highly suitable as a pharmaceutical agent.

The present invention is further illustrated by the following Reference Example, Example and Test Examples, but is not limited thereto. Unless otherwise indicated, all percents, ratios, parts and the like are by weight.

## REFERENCE EXAMPLE

Preparation of glyco-related antigen TCA

To 100 to 120 g of cancer cells obtained from human gastric cancer, which had been stored in a frozen state at -80°C, approximately 480 ml of a 0.01 M phosphate-buffered physiological saline solution (PBS; pH 7.2) was added, and the cells were homogenized in ice water. The solution thus obtained was poured into

4

six tubes of an angle type centrifugator, and ultra-centrifuged at 100,000 g with the use of an RP 42 angle type roter (37,000 rpm, at 4°C) for an hour. The supernatant (the lst supernatant) was transferred to a separate container by decantation and stored in a frozen state at -80°C. On the other hand, the precipitate (the lst precipitate) was collected and weighed. 180 ml of a 0.01 M tris hydrochloride buffer (pH 7.6; E-Buffer) containing 2% of Triton X-100 was added to approximately 20 g of the lst precipitate, and the resulting mixture was stirred with a stirrer for five minutes and then homogenized in ice water. The resulting solution was poured into six tubes of a swing type centrifugator and ultracentrifuged at 80,000 g with the use of an RPS 27-2 swing type roter (26,000 rpm, at 4°C) for 1.5 hour. The uppermost fat phase was removed by sucking with an aspirator to obtain a supernatant (the 2nd supernatant). Another 20 g portion of the lst precipitate was subjected to the same treatment to obtain the 2nd supernatant. The precipitates (the 2nd precipitate) were combined and stored in a frozen state at -80°C. These 2nd supernatants were added to a PNA-Sepharose column equilibrated with the E-Buffer containing 2% of Triton X-100 in a cold chamber at 4°C with the use of a peristaltic pump over a period of approximately 24 to 36 hours. Then, the column was washed with the E-Buffer containing 2% of Triton X-100 over a period of 12 to 24 hours, then with the E-Buffer containing 0.5% of Triton X-100 over a period of 12 to 24 hours and finally with the E-Buffer containing 0.1% of Triton X-100 over a period of 12 to 24 hours. The column was eluted with approximately 70 ml of the E-Buffer containing 0.2 M of lactose and 0.1% of Triton X-100 over a period of 8 hours, and the eluate was collected in 100-drop portions by a fraction collecter. The absorbance of each fraction at 280 nm was measured, and the fractions showing a peak of proteins were combined. The combined fractions were sterilized by filtering through a membrane, introduced into a Cellophane dialysis tube and dialyzed 12 times against 5 ℓ of physiological saline solution in a cold chamber at 4°C for four days. The glyco-related antigen TCA thus obtained was stored in a frozen state at -80°C.

## EXAMPLE 1

Preparation of JI-36

To approximately 75 ml of the glyco-related antigen TCA obtained above containing 30 mg of proteins was added 20 ml of a 2.5 M tris hydrochloride buffer. Further, 1 g of sodium dodecylsulfate (SDS) was added thereto and dissolved therein. After subjecting the resulting mixture to an ultrasonic treatment at 25°C for 15 minutes, 5 ml of a 0.5 M tris hydrochloride buffer in which 200 mg of DTT was dissolved was added thereto. After replacing with nitrogen, the mixture was sealed and allowed to stand at 25°C for 12 hours. Then, 5 ml of a 0.5 M tris hydrochloride buffer in which 300 mg of IAA had been dissolved was added thereto, and the resulting mixture was allowed to stand at 25°C for 1 to 2 hours. After adding 1 ml of a 10% Triton X-100 solution, the mixture was sterilized by filtering through a membrane and dialyzed twice against 5 ℓ of PBS containing 0.1% of Triton X-100 at room temperature for three days. Then, the dialyzed mixture was added to a PNA-Sepharose column equilibrated with PBS containing 0.1% of Triton X-100 in a cold chamber at 4°C through a peristaltic pump over a period of approximately 16 to 24 hours. Subsequently, the column was washed with approximately 1 ℓ of PBS containing 0.1% of Triton X-100 over a period of 48 hours and then with approximately 500 ml of PBS containing 0.01% of Tween over a period of 24 hours. The column was eluted with approximately 70 ml of PBS containing 0.2 M of lactose and 0.01% of Tween 80 over a period of 8 hours, and the eluate was collected in 200-drop portions by a fraction collecter. The absorbance of each fraction at 280 nm was measured and fraction showing a peak of proteins were combined. The combined fractions were sterilized by filtering through a membrane, introduced into a Cellophane dialysis tube and dialyzed 12 times against 5 ℓ of physiological saline solution containing 0.01% of Tween 80 in a cold chamber at 4°C. for four days. After sterilizing by filtering through a membrane, the thus obtained JI-36 was stored at 4°C.

## TEST EXAMPLE 1

Analysis of Sugar Chain Structure

The JI-36 was labeled with $^3$H by the galactose oxidase NAB$^3$H$_4$ method and the sugar chain structure thereof was analyzed by sugar composition analysis, methylation analysis, oxidation with periodic acid, glycosidase digestion and identification of the reduced terminal sugar. As a result, it was found that it has the following two sugar chain structures:

Gal$\beta$1 → 3GalNac- and

Gal$\beta$1 → 3GlcNac$\beta$1 → 6(Gal$\beta$1 → 3)GalNAc-.


TEST EXAMPLE 2


Sugar Protein Ratio

The procedure of Example 1 was repeated to thereby obtain 43 lots of JI-36 in total. The sugar/protein ratio of each lot was determined.

The neutral sugar content was determined by the phenol-sulfuric acid method using glucose as a standard material. The amino sugar content was determined by the Morgan-Elson reaction method using N-acetylglucosamine as a standard material. The protein content was determined by the Lowry method using human serum albumin as a standard material.

The sugar protein ratio calculated by the formula:

$$\frac{\text{neutral sugar content} + \text{amino sugar content}}{\text{protein content}}$$

of the 43 lots was found to be 1.3±0.3 (mean ± standard deviation).


TEST EXAMPLE 3


Sugar Composition

The 43 lots of JI-36 were subjected to methanolysis according to the method reported in Clamp et al., Biochem. Biophys. Acta., 222, pp. 339-347 (1970), and then N-acetylated again to obtain a trimethylsilyl derivative. The resulting product was analyzed by gas chromatography using a gas chromatogram GC-2D (PF) (a product of Shimazu Seisakusho Co., Ltd.) and using a column HiCap-CBP-M-25-0.25 (0.2 mm (di.)-x25 cm) (a product of Shimazu Seisakusho Co., Ltd.). Table 1 shows the results of the analysis on the 43 lots.

Table 1

| | Molar Ratio (%) | |
| --- | --- | --- |
| Sugar Components | Mean | Standard Deviation |
| Fuc | 6.19 | 1.52 |
| Man | 6.59 | 0.78 |
| Gal | 37.97 | 2.66 |
| GalNAc | 14.23 | 2.12 |
| GlcNAc | 30.60 | 3.45 |
| Sia | 4.60 | 1.45 |

## TEST EXAMPLE 4

UV Absorption Spectrum

The UV absorption spectra at 200 nm to 600 nm of three lots of JI-36 were measured with a Hitachi Spectrophotometer 220A (a product of Hitachi Co., Ltd.)

As a result, each of the lot showed the maximum absorption in the range of from 270 to 280 nm.

## TEST EXAMPLE 5

Disc-Polyacrylamide Gel Electrophoresis (Disc-PAGE)

Two lots of JI-36 were subjected to Disc-PAGE according to the method reported in B. Davis et al., Ann. N.Y. Acad. Sci., 121. pp. 404 (1964)). That is, a sample containing 40 $\mu$g of proteins (in the case of protein-staining) or 40 $\mu$g of sugars (in the case of sugar-staining) was added to a 3.5% polyacrylamide gel and electrophoretically migrated at a constant voltage of 125 V until the colorant to be monitored reached the bottom of the gel. Coomassie Brilliant Blue (CBB) was used for the protein-staining and periodic acid-Schiff's reagent (PAS) was used for the sugar-staining. In both of the stainings and in the reproduced lot, JI-36 showed an Rf value of 0.3 or below.

## TEST EXAMPLE 6

Polyacrylamide Gel Electrophoresis (PAGE)

Three lots of JI-36 were subjected to PAGE according to the method reported in M. Lasky et al., Electrophoresis, pp. 195-210 (1978).

That is, a sample containing 40 $\mu$g of proteins (in the case of protein-staining) or 40 $\mu$g of sugars (in the case of sugar-staining) was added to a 2 to 16% polyacrylamide gradient gel (manufactured by Pharmacia) and electrophoretically migrated at a constant voltage of 150 V for 16 hours (2400 Vh). The protein-staining and sugar-staining were effected in the same manner as those described above. In both stainings, each lot migrated into a region which could not determined with the use of a known standard high molecular weight protein, indicating that the protein was migrated into a region over the thyroglobulin region (669,000).

## TEST EXAMPLE 7

Solubility

The solubility of the JI-36 was examined by using three lots of JI-36. That is, a sample was diluted 20-fold with a physiological saline solution and that containing 100 $\mu$g/ml of Tween 80 to thereby obtain final concentrations of Tween 80 of 5 $\mu$g/ml and 100 $\mu$g/ml. A portion of each of the solution thus obtained was used as a control solution, and the remaining portion of the solution was ultracentrifuged at 28,000 rpm (100,000 g) for two hours. After the centrifugation, the titer of each supernatant was determined.

The titer was determined by the following enzyme-lectin assay. That is, a microplate was coated with PNA to obtain a solid phase, and the sample solution was added to the microplate, incubated and then reacted with peroxidase-labeled PNA. Thus, the titer was determined based on the PNA-binding activity of the sample.

A 1 μg ml solution of asialoglycophorin was used as a standard, and the PNA-binding activity of the solution was referred to as 3000 U/ml. Thus, the titer of the sample (U/ml) was calculated. The results obtained are shown in Table 2 below.

Table 2

| Sample | Tween 80 Concentration | Treatment | Titer (x10³ U/ml) | % Remaining |
|---|---|---|---|---|
| | (μg/ml) | | | (%) |
| Lot 44 | 5 | control | 1.81 | - |
| | | centrifuged | 1.66 | 92 |
| | 100 | control | 1.72 | - |
| | | centrifuged | 1.60 | 93 |
| Lot 53 | 5 | control | 1.83 | - |
| | | centrifuged | 1.73 | 95 |
| | 100 | control | 1.83 | - |
| | | centrifuged | 1.76 | 96 |
| Lot 66 | 5 | control | 0.82 | - |
| | | centrifuged | 0.89 | 109 |
| | 100 | control | 0.90 | - |
| | | centrifuged | 0.80 | 89 |

The results shown in Table 2 indicates that each lot showed a high solubility

## TEST EXAMPLE 8

Reactivity with Antibody

Each of 43 lots of JI-36 was introduced into wells of an EIA plate (96 well flat bottomed titer plate No. 655101 manufactured by Greiner) and solidified to obtain a solid phase. Each of the solidified JI-36 was reacted with monoclonal antibodies, and then the antibodies thus bound was assayed by using a peroxidase-labeled antimouse IgM antibody (manufactured by CAPDEL) to examine the reactivity of JI-36 with each monoclonal antibody.

As a result, it was confirmed that the JI-36 is reactive with the monoclonal antibodies FH-2, ACFH-18, FH-6 and AH-6 in all of the 43 lots.

## TEST EXAMPLE 9

Agglutination

JI-36 was subjected to freezing-thawing treatment five times and then to gel-filtration (TSKG 5000 PWXL, manufactured by Tosoh Corporation). Then, the elution pattern was detected based on the protein absorption and the titer described above. No change was observed in the elution pattern before and after the above treatment.

No decrease was observed in the biological activity of the JI-36 before and after the above treatment, when the killer cells were prepared and the cytotoxic activity thereof was examined according to the Test Example 7 of Japanese Patent Application (OPI) No. 1420/84. Furthermore, no significant difference was observed in the average particle size of the JI-36 particles before and after the above treatment, when determined with a scattering photometer DLS-700 (manufactured by Otsuka Denshi K.K.).

## TEST EXAMPLE 10

Induction of Cytotoxic Lymphocytes (killer cells) with JI-36

Lymphocytes were prepared in a conventional manner as described in Immunological Test Procedures, Vol. X, 3305-3316, ed. Immn. Soc. of Japan). That is, silica (KAC-2; manufactured by JIMRO) was added to the peripheral blood of a healthy human subject to conduct phagocytosis. Then, a nonphagocytic lymphocyte fraction was obtained by density gradient centrifugation. After removing adherent cells with a nylon wool column, a concentrated large granular lymphocyte (LGL) fraction, which corresponded to a low specific gravity fraction in the density gradient centrifugation, was obtained.

To the resulting LGL fraction, JI-36 was added to obtain a final concentration of 0 U/ml (control), 2 U/ml, 20 U/ml or 200 U/ml. Each mixture was incubated in a conventional manner for 60 minutes to thereby induce killer cells (effecter cells: E).

The cancer cytotoxicity (killer activity) of the E cells thus induced was determined according to a process described in Japanese Patent Application (OPI) No. 1420/84.

Regarding target cells (T), a cell strain BT-1 originating from Burkitt's lymphoma was used as PNA binding sugar chain positive cells and a cell strain MOLT-4 originating from T leukemia was used as PNA-binding sugar chain weekly positive cells. The cytotoxicity was determined at an E/T ratio of 10:1 for an E/T reaction period of 14 to 18 hours.

The killer activity was expressed in the loss (%) of T determined from the cytogram fraction (EPICS C; Coulter Corp., X axis: side scattering light, Y axis: front scattering light) by flow cytometry as described in "Determination of NK activity by Flow Cytometry", 19th Report of Japan Automatic Clinical Examination Soc., Vol. 12, p. 575 (1987).

The results obtained are shown in Table 3.

Table 3

| | E | | | |
|---|---|---|---|---|
| T | Control | 2 U/ml group | 20 U/ml group | 200 u/ml group |
| BT-1 | 10.8±7.9 | 13.2±7.6 | 17.5±7.1 | 15.9±8.4 |
| MOLT-4 | 46.4±18.7 | 50.9±19.0 | 51.6±19.1 | 48.8±20.6 |
| Killer activity (%): mean±S.D. (n = 7). | | | | |

Table 3 indicates that the JI-36 of the present invention can effectively induce killer cells.

Each of the 20 U/ml group of BT-1, and the 2 U/ml and 20 U/ml groups of MOLT-4 showed a significant difference (P<0.05) as determined by the Wilcoxon's U test.

## TEST EXAMPLE 11

The antiarthritic activity of JI-36 was evaluated in the collagen-induced arthritis which is generally used as animal model of chronic articular rheumatism. The collagen arthritis was induced according to the method of Trentham et al., J. Exp. Med., 146, 857, 1977. Twelve female Wistar-Lewis rats (7.5 week-old) (available from Japan Charles River) were sensitized with 1 ml of an emulsion of 0.5 mg Type II collagen derived from bovine catilage (available from Sigma) in Freund incomplete adjuvant (available from Difco) by intracutaneous injection on the back. The condition of arthritis was scored by the blind method after the 13th day from the collagen sensitization, based on the arthritis evaluation standard of 3 ranks in forefoot and 14 ranks in post peduncle.

The administration of JI-36 was started from 7 days before the collagen sensitization at a dose of 0.178 U/body (0.2 ng/body) in a physiological soline solution containing Tween 80 (5 μg/ml) and JI-36 (2 ng/ml) in a volume of 0.1 ml/body according to a schedule of (1) 3 consecutive days' administration and (2) 4 consecutive days' non-administration per week. The results obtained are shown in Figure as compared with the control group which received only a solvent (a physiological saline solution containing 5 μg/ml of Tween

80).

As is noted from Figure, the generation of arthritis and the increase in the arthritic condition were observed from the 13th day after the collagen sensitization in both the test and control group, but a significant reduction (P<0.05) in the athritic conditions was observed in the rat group which received JI-36 as compared with the control group, when determined by the Wilcoxon U Test.

While the invention has been described in detail and with reference to specific embodiments thereof, it will be apparent to one skilled in the art that various changes and modifications can be made therein without departing from the spirit and scope thereof.

## Claims

1. A glycoprotein JI-36 which is obtained from a cancer cell membrane component and has the following properties:

(1) it has sugar chains of a Gal$\beta$1→3 GalNAc structure and a Gal$\beta$1→4 GlcNAc$\beta$1→6 (Gal$\beta$1→3)-GalNAc structure;

(2) it shows a glycoprotein ratio of (neutral sugar content + aminosugar content)/protein content of 1.3±0.3, wherein the neutral sugar content was determined by the phenol-sulfuric acid method, the aminosugar content was determined by the Morgan-Elson reaction and the protein content is determined by the Lowry method;

(3) it comprises, as sugar components, Gal and GlcNAc as the main components together with Fuc, Man, GalNAc and Sia;

(4) It shows the maximum absorptions at 270 to 280 nm in the UV absorption spectrum thereof; and

(5) it induces an immune response specific to cancers.

2. A process for preparing a glycoprotein JI-36 as set forth in claim 1 which comprises reducing a glyco-related antigen TCA derived from a cancer cell membrane component and reducing the glyco-related antigen with an SH group-modifying reagent.

3. Pharmaceutical composition comprising a glycoprotein JI-36 as set forth in claim 1 as the active ingredient and a pharmaceutically acceptable carrier.

4. Use of a glycoprotein JI-36 as set forth in claim 1 for making a medicament for the treatment and prevention of cancers.

5. Use of a glycoprotein JI-36 as set forth in claim 1 for making a medicament effective in the treatment and prevention of immunopathy diseases such as rheumatoid arthritis.

Claims for the following Contracting State: ES

1. A process for preparing a glycoprotein JI-36 which is obtained from a cancer cell membrane component and has the following properties:

(1) it has sugar chains of a Gal$\beta$1→3 GalNAc structure and a Gal$\beta$1→4 GlcNAc$\beta$1→6 (Gal$\beta$1→3)-GalNAc structure;

(2) it shows a glycoprotein ratio of (neutral sugar content + aminosugar content)/protein content of 1.3±0.3, wherein the neutral sugar content was determined by the phenol-sulfuric acid method, the aminosugar content was determined by the Morgan-Elson reaction and the protein content is determined by the Lowry method;

(3) it comprises, as sugar components, Gal and GlcNAc as the main components together with Fuc, Man, GalNAc and Sia;

(4) It shows the maximum absorptions at 270 to 280 nm in the UV absorption spectrum thereof; and

(5) it induces an immune response specific to cancers, which comprises reducing a glyco-related antigen TCA derived from a cancer cell membrane component and reacting the glyco-related antigen with an SH group-modifying reagent.

2. Use of a glycoprotein JI-36 as set forth in claim 1 for making a medicament for the treatment and prevention of cancers.

3. Use of a glycoprotein JI-36 as set forth in claim 1 for making a medicament effective in the treatment and prevention of immunopathy diseases such as rheumatoid arthritis.

## DOCUMENTS CONSIDERED TO BE RELEVANT

| Category | Citation of document with indication, where appropriate, of relevant passages | Relevant to claim | CLASSIFICATION OF THE APPLICATION (Int. Cl.4) |
|---|---|---|---|
| X | JOURNAL OF BIOCHEMISTRY, vol. 102, no. 3, 1987, pages 657-664; N. SHIMODA et al.: "Carbohydrate moieties of peanut agglutinin receptors isolated from human gastric cancer cells" * Page 664: "Discussion" * | 1-3 | A 61 K 37/02 // A 61 K 35/38 |
| A | CHEMICAL ABSTRACTS, vol. 99, no. 15, 10th October 1983, page 453, abstract no. 120127n, Columbus, Ohio, US; J. FISCHER et al.: "Lectin binding properties of glycoproteins in cells of normal gastric mucosa and gastric cancers: a comparative histochemical and biochemical study", & CANCER DETECT. PREV 1983, 6(1-2), 137-47 * Abstract * | | |

TECHNICAL FIELDS
SEARCHED (Int. Cl.4)

A 61 K
C 07 K

The present search report has been drawn up for all claims

| Place of search | Date of completion of the search | Examiner |
|---|---|---|
| THE HAGUE | 09-02-1989 | TURMO Y BLANCO C.E. |

EPO FORM 1503 03.82 (P0401)